# EUROPEAN PATENT APPLICATION

(11) **EP 2 537 459 A1**
(43) Date of publication of application: **26.12.2012**
(21) Application number: 12004624.8
(22) Date of filing: 20.06.2012
(51) Int. Cl.: A61B 1/05, A61B 1/00

(54) **Electronic endoscopic apparatus**

(30) Priority: 20.06.2011 JP 2011136409
(71) Applicant: OLYMPUS CORPORATION, Shibuya-ku Tokyo (JP)
(72) Inventor: Azuma, Motoo, Tokyo (JP); Takizawa, Kazuhiro, Tokyo (JP); Tanaka, Satoshi, Tokyo (JP); Sato, Takayuki, Tokyo (JP); Kobayashi, Naruyasu, Tokyo (JP); Kotoda, Kaoru, Tokyo (JP); Nishimura, Hisashi, Tokyo (JP)
(74) Representative: Gunzelmann, Rainer

(57) **Abstract**

An imaging device (114) is installed on a scope distal portion (110), and captures an image based on an imaging clock. An image processor portion (120) performs image processing on the image captured by the imaging device (114), and displays the corresponding image based on a display clock on a monitor. A scope cable portion (130) transmits data between the scope distal portion (110) and the image processor portion (120). A clock oscillator (111) generates a master clock. A first multiplying/dividing circuit (112) multiplies and/or divides the master clock by (natural number/natural number), and generates a transmission clock whose frequency is lower than that of the imaging clock and is (natural number) times the frequency of a vertical synchronization signal.

## Description

### BACKGROUND OF THE INVENTION

### Field of the Invention

The present invention relates to an electronic endoscopic apparatus.

### Description of Related Art

With the recent advancement of semiconductor technology, solid-state imaging devices such as charge-coupled devices (CCDs) or complementary metal oxide semiconductor (CMOS) sensors are moving toward higher pixels. Electronic endoscopic apparatuses are no exception to this trend, and solid-state imaging devices mounted in the electronic endoscopic apparatuses are moving toward higher pixels. With the trend toward higher pixels in the solid-state imaging devices, a frequency of a clock signal required for image processing is also increasing, and various phenomena take place. For example, the electronic endoscopic apparatuses have a structure in which a distal end of a scope on which the solid-state imaging device is mounted is separated from an image processor performing image processing. For this reason, a transmission line between the solid-state imaging device and the image processor may be subjected to signal degradation. Further, when the frequency of the clock signal becomes high, the signal degradation has a much greater influence on the transmission line between the solid-state imaging device and the image processor. Further, because of the high-frequency signal traveling through the transmission line between the solid-state imaging device and the image processor, leakage of electromagnetic waves also becomes more significant.

Such an electronic endoscopic apparatus is proposed in Japanese Unexamined Patent Application, First Publication No. 2001-275956. FIG. 8 is a block diagram showing a configuration of an electronic endoscopic apparatus known in the related art. An example shown is configured so that a waveform smoothing circuit 916 is inserted into an output stage of an electronic scope 900, and thereby high-frequency noise released between the electronic scope 900 and a processor device 950 is suppressed.

Japanese Unexamined Patent Application, First Publication No. 2001-275956 contains no teaching in terms of synchronization between an electronic scope (endoscopic scope) and a monitor (image processing processor). Since a solid-state imaging device having various angles of view depending on a target to be observed and use is mounted on the endoscopic scope, an operating frequency and an angle of view are different according to the endoscopic scope. Accordingly, to display a moving image captured by the endoscopic scope on the monitor, frequency conversion adapted to a synchronization signal of the monitor is required. Further, the endoscopic scope captures the moving image at a timing based on an imaging clock, while the monitor displays the moving image at a timing based on a display clock.

When the frequency conversion is performed, depending on a relation between the imaging clock and the display clock, there is a subtle difference between a cycle in which the electronic scope captures an image of one frame and a cycle in which the monitor displays an image of one frame. As such, the two cycles are gradually shifted in phase. Thus, when the phase shift is accumulated, and when the phase shift between the two cycles exceeds a cycle of one frame, a phenomenon called "passing" or "frame dropping" takes place.

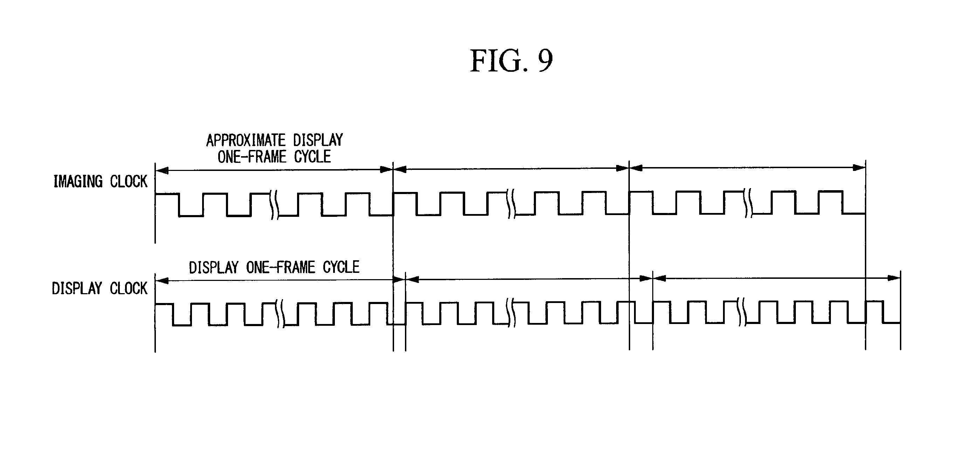

FIG. 9 schematically shows a relation between a one-frame cycle based on an imaging clock and a one-frame cycle based on a display clock. As shown in FIG. 9, a frequency of the imaging clock and a frequency of the display clock are different from each other. For this reason, the one-frame cycle based on the imaging clock and the one-frame cycle based on the display clock are slightly misaligned. A slight shift is present within the one-frame. However, as shown in FIG. 9, the shift accumulates with the lapse of time. Thus, when the shift exceeds a one-frame cycle, a phenomenon called "passing" or "frame dropping" takes place.

On the other hand, even in a monitor side, rapidization is progressing according to the higher definition, and input of signals into the monitor is required to meet strict timing rules. Even if the one-frame cycle for imaging can completely match that for displaying, when a synchronization signal is generated based on the clock of the endoscopic scope which does not comply with television standards, the monitor has a possibility of not carrying out normal display.

### SUMMARY OF THE INVENTION

The present invention provides an electronic endoscopic apparatus capable of securing synchronization between imaging and displaying.

According to a first aspect of the present invention, an electronic endoscopic apparatus includes: an imaging device that is installed on a scope distal portion and captures an image based on an imaging clock; an image processor portion that performs image processing on the image captured by the imaging device and displays the corresponding image on a monitor based on a display clock; a scope cable portion that transmits data between the scope distal portion and the image processor portion; a clock oscillator that generates a master clock; and a multiplying/dividing circuit that multiplies and/or divides the master clock by (natural number/natural number) and generates a transmission clock whose frequency is lower than that of the imaging clock and is (natural number) times the frequency of a vertical synchronization signal.

According to a second aspect of the present invention, the electronic endoscopic apparatus further includes a second multiplying/dividing circuit that multiplies and/or divides the transmission clock by (natural number/natural number) and generates a necessary frequency. The imaging clock or the display clock is generated from the master clock.

According to a third aspect of the present invention, in the electronic endoscopic apparatus, the clock oscillator is installed on the scope distal portion. The multiplying/dividing circuit is installed on the scope distal portion. The clock oscillator and the multiplying/dividing circuit generate the imaging clock and the transmission clock. The second multiplying/dividing circuit is installed on the image processor portion, and generates the display clock.

According to a fourth aspect of the present invention, in the electronic endoscopic apparatus, the multiplying/dividing circuit multiplies and/or divides the master clock by (natural number/natural number), and generates the imaging clock. The second multiplying/dividing circuit multiplies and/or divides the transmission clock by (natural number/natural number), and generates the display clock.

According to a fifth aspect of the present invention, the electronic endoscopic apparatus includes a display synchronization signal generating unit that generates a display vertical synchronization signal for displaying the image from the display clock. The scope cable portion transmits the transmission clock and the display vertical synchronization signal from the image processor portion to the scope distal portion.

According to a sixth aspect of the present invention, the electronic endoscopic apparatus includes an imaging synchronization signal generating unit that generates an imaging vertical synchronization signal for capturing the image from the imaging clock at a timing preceding a timing at which the display vertical synchronization signal is generated by at least a time that is the sum of a transmission delay time of the scope cable portion and a processing time at the image processor portion.

According to a seventh aspect of the present invention, the electronic endoscopic apparatus includes an imaging synchronization signal generating unit that generates an imaging vertical synchronization signal for capturing the image from the imaging clock. The scope cable portion transmits the transmission clock and the imaging vertical synchronization signal from the scope distal portion to the image processor portion.

According to an eighth aspect of the present invention, the electronic endoscopic apparatus includes a display synchronization signal generating unit that generates a display vertical synchronization signal for displaying the image from the display clock at a timing delaying from a timing at which the imaging vertical synchronization signal is generated by at least a time that is the sum of a transmission delay time of the scope cable portion and a processing time at the image processor portion.

According to a ninth aspect of the present invention, in the electronic endoscopic apparatus, the clock oscillator is installed on the image processor portion. The multiplying/dividing circuit is installed on the image processor portion. The clock oscillator and the multiplying/dividing circuit generate the display clock and the transmission clock. The second multiplying/dividing circuit is installed on the scope distal portion, and generates the imaging clock.

According to a tenth aspect of the present invention, in the electronic endoscopic apparatus, the multiplying/dividing circuit multiplies and/or divides the master clock by (natural number/natural number), and generates the display clock. The second multiplying/dividing circuit multiplies and/or divides the transmission clock by (natural number/natural number), and generates the imaging clock.

According to an eleventh aspect of the present invention, the electronic endoscopic apparatus includes a display synchronization signal generating unit that generates a display vertical synchronization signal for displaying the image from the display clock. The scope cable portion transmits the transmission clock and the display vertical synchronization signal from the image processor portion to the scope distal portion.

According to a twelfth aspect of the present invention, the electronic endoscopic apparatus includes an imaging synchronization signal generating unit that generates an imaging vertical synchronization signal for capturing the image from the imaging clock at a timing preceding a timing at which the display vertical synchronization signal is generated by at least a time that is the sum of a transmission delay time of the scope cable portion and a processing time at the image processor portion.

According to a thirteenth aspect of the present invention, the electronic endoscopic apparatus includes an imaging synchronization signal generating unit that generates an imaging vertical synchronization signal for capturing the image from the imaging clock. The scope cable portion transmits the transmission clock and the imaging vertical synchronization signal from the scope distal portion to the image processor portion.

According to a fourteenth aspect of the present invention, the electronic endoscopic apparatus includes a display synchronization signal generating unit that generates a display vertical synchronization signal for displaying the image from the display clock at a timing delaying from a timing at which the imaging vertical synchronization signal is generated by at least a time that is the sum of a transmission delay time of the scope cable portion and a processing time at the image processor portion.

According to a fifteenth aspect of the present invention, in the electronic endoscopic apparatus, the imaging vertical synchronization signal and the display vertical synchronization signal are generated so as to have the same frequency.

According to the electronic endoscopic apparatus, the imaging device is installed on the scope distal portion and captures the image based on the imaging clock. Further, the image processor portion performs image processing on the image captured by the imaging device and displays the corresponding image on the monitor based on the display clock. Also, the scope cable portion transmits data between the scope distal portion and the image processor portion. Further, the clock oscillator generates the master clock. In addition, the multiplying/dividing circuit multiplies and/or divides the master clock by (natural number/natural number), and generates the transmission clock whose frequency is lower than that of the imaging clock and is (natural number) times the frequency of the vertical synchronization signal. Thereby, the electronic endoscopic apparatus can multiply and/or divide the master clock by (natural number/natural number), and operate using the transmission clock whose frequency is lower than that of the imaging clock and is (natural number) times the frequency of the vertical synchronization signal. For this reason, it is possible to secure the synchronization between the imaging and the displaying.

### BRIEF DESCRIPTION OF THE DRAWINGS

FIG. 1 is a block diagram showing a configuration of an electronic endoscopic apparatus according to a first embodiment of the present invention.
FIG. 2 is a block diagram showing a configuration of an electronic endoscopic apparatus according to a second embodiment of the present invention.
FIG. 3 is a block diagram showing a configuration of an electronic endoscopic apparatus according to a third embodiment of the present invention.
FIG. 4 is a block diagram showing a configuration of an electronic endoscopic apparatus according to a fourth embodiment of the present invention.
FIG. 5 is a block diagram showing a configuration of an electronic endoscopic apparatus according to a modified example.
FIG. 6 is a block diagram showing a configuration of an electronic endoscopic apparatus according to a modified example.
FIG. 7 is a block diagram showing a configuration of an electronic endoscopic apparatus according to a modified example.

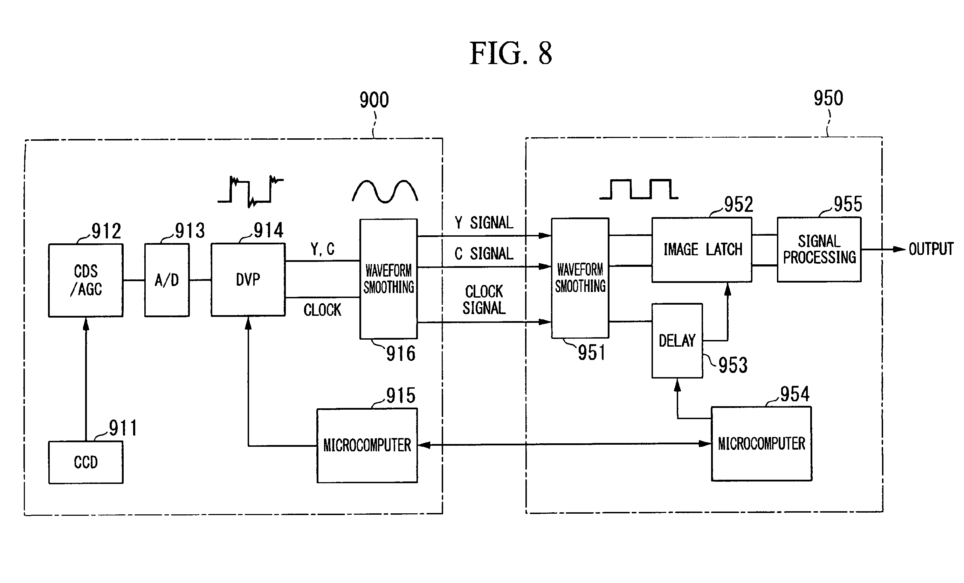

FIG. 8 is a block diagram showing a configuration of an electronic endoscopic apparatus that is known in the related art.
FIG. 9 is a schematic diagram showing a relation between a one-frame cycle based on an imaging clock and a one-frame cycle based on a display clock.

### DETAILED DESCRIPTION OF THE INVENTION

### (First Embodiment)

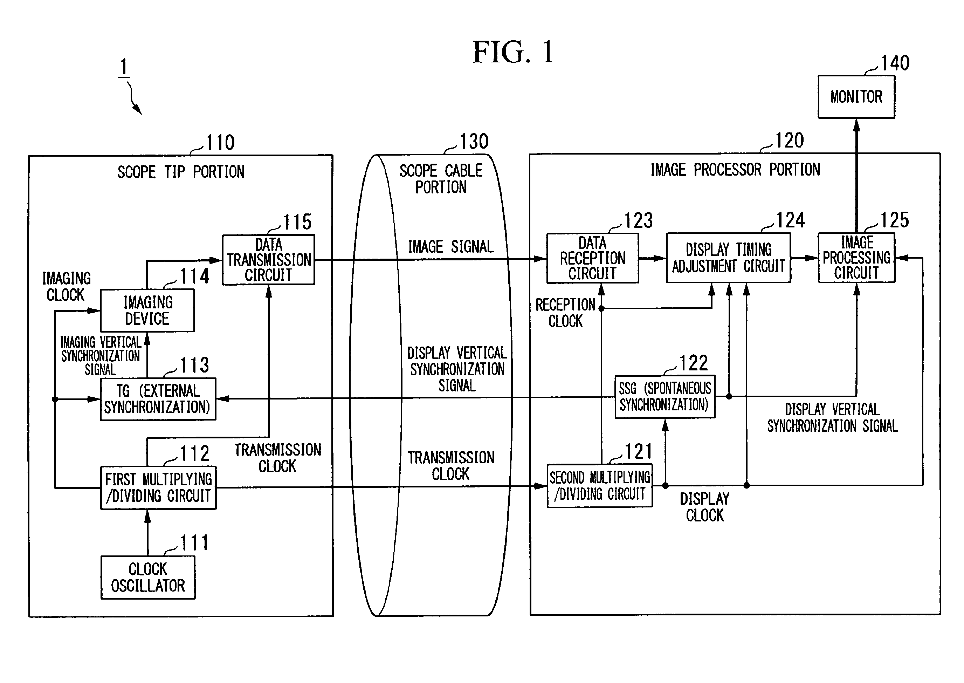

Hereinafter, a first embodiment of the present invention will be described with reference to the drawings. FIG. 1 is a block diagram showing a configuration of an electronic endoscopic apparatus in the present embodiment. In the example shown in FIG. 1, the electronic endoscopic apparatus 1 includes a scope distal portion 110, an image processor portion 120, a scope cable portion 130, and a monitor 140. The scope distal portion 110 is inserted into a living body, and captures an image in the living body. The image processor portion 120 performs image processing of converting an image signal transmitted from the scope distal portion 110 in a pattern in which the image signal is displayed on the monitor 140, and displaying the converted result on the monitor 140. The scope cable portion 130 performs data transmission between the scope distal portion 110 and the image processor portion 120, for example, transmits the image signal captured by the scope distal portion 110 to the image processor portion 120 outside the living body. The monitor 140 is a display device such as a liquid crystal display, and displays an image (moving image).

The scope distal portion 110 includes a clock oscillator 111, a first multiplying/dividing circuit (multiplying/dividing circuit) 112, a timing generator (TG) (imaging synchronization signal generator) 113, an imaging device 114, and a data transmission circuit 115.

The clock oscillator 111 is an oscillator such as a crystal, and generates a master clock. The first multiplying/dividing circuit 112 multiplies and/or divides the master clock by (natural number/natural number), and generates an imaging clock for driving the imaging device 114 and the TG 113, a transmission clock to be fed to the data transmission circuit 115, and a transmission clock to be transmitted to the image processor portion 120. Further, the (natural number/natural number) may be predetermined, or may be adapted to be able to be arbitrarily set. The transmission clock generated by the first multiplying/dividing circuit 112 is transmitted to the image processor portion 120 via the scope cable portion 130.

A display vertical synchronization signal transmitted from the image processor portion 120 is input to the TG 113. Further, the TG 113 generates a variety of control signals, which include an imaging vertical synchronization signal for driving the imaging device 114, from the imaging clock generated by the first multiplying/dividing circuit 112, in a cycle that is completely identical to that of the input display vertical synchronization signal at a timing that is ahead of a timing at which the display vertical synchronization signal is generated by at least a time that is the sum of the transmission delay time of the scope cable portion 130 and the processing time at the image processor portion 120. Thereby, in consideration of the delay, it is possible to secure synchronization between imaging and displaying.

The imaging device 114 is operated by the imaging clock generated by the first multiplying/dividing circuit 112, and outputs an image signal corresponding to incident light at a timing based on the imaging vertical synchronization signal generated by the TG 113 (captures a one-frame image). The data transmission circuit 115 converts a pattern of the image signal output by the imaging device 114 into a pattern in which the scope cable portion 130 can transmit the signal using the transmission clock generated by the first multiplying/dividing circuit 112. For example, such conversion is performed by a well-known method of conducting conversion from a pattern in which a pixel value is expressed in a multi-bit parallel pattern to a serial form, conducting 8b/10b conversion, and conducting conversion in a differential form. Then, the data transmission circuit 115 transmits the converted image signal to the image processor portion 120 via the scope cable portion 130.

The image processor portion 120 includes a second multiplying/dividing circuit 121, a sync signal generator (SSG) (display synchronization signal generator) 122, a data reception circuit 123, a display timing adjustment circuit 124, and an image processing circuit 125.

The second multiplying/dividing circuit 121 receives the transmission clock transmitted from the scope distal portion 110. Then, the second multiplying/dividing circuit 121 multiplies and/or divides the received transmission clock by (natural number/natural number), generates a reception clock having a necessary frequency in order to receive the image signal transmitted from the scope distal portion 110, and outputs the generated reception clock to the data reception circuit 123 and the display timing adjustment circuit 124. Further, the second multiplying/dividing circuit 121 multiplies and/or divides the transmission clock transmitted from the scope distal portion 110 by (natural number/natural number), generates a display clock having a necessary frequency in order to display an image, which is based on the image signal transmitted from the scope distal portion 110, on the monitor 140, and outputs the generated display clock to the SSG 122, the display timing adjustment circuit 124, and the image processing circuit 125. Further, the (natural number/natural number) may be predetermined, or may be adapted to be able to be arbitrarily set. Further, in the first multiplying/dividing circuit 112 and the second multiplying/dividing circuit 121, each magnification (natural number/natural number) of the multiplication and/or division may be either equal or different.

The SSG 122 generates a display vertical synchronization signal indicating a timing at which the image based on the image signal is displayed on the monitor 140 as well as a variety of timing signals from the display clock input from the second multiplying/dividing circuit 121, and outputs the generated signals to the display timing adjustment circuit 124 and the image processing circuit 125. Further, the display vertical synchronization signal generated by the SSG 122 is transmitted to the scope distal portion 110 via the scope cable portion 130. The display vertical synchronization signal transmitted to the scope distal portion 110 is input to the TG 113.

The data reception circuit 123 is operated by the reception clock, receives the image signal transmitted from the scope distal portion 110 via the scope cable portion 130, and outputs the received image signal to the display timing adjustment circuit 124. The display timing adjustment circuit 124 puts the image signal input from the data reception circuit 123 in a frequency of the display clock generated by the second multiplying/dividing circuit 121, and outputs the resultant image signal to the image processing circuit 125. The image processing circuit 125 is operated by the display clock, performs image processing on the input image signal, and causes the monitor 140 to displays an image (one-frame image) based on the image signal at a timing based on the display vertical synchronization signal generated by the SSG 122.

Next, an operation of the electronic endoscopic apparatus 1 in the present embodiment will be described. In the present embodiment, the scope distal portion 110 generates a master clock, multiplies and/or divides the master clock by (natural number/natural number), and generates an imaging clock and a transmission clock. Further, the scope distal portion 110 generates an imaging vertical synchronization signal from the imaging clock so as to be synchronized with the display vertical synchronization signal transmitted from the image processor portion 120, and captures a one-frame image at a timing based on the imaging vertical synchronization signal. Further, the scope distal portion 110 transmits the generated transmission clock to the image processor portion 120.

On the other hand, the image processor portion 120 generates a display vertical synchronization signal from the transmitted transmission clock, and displays a one-frame image at a timing based on the display vertical synchronization signal. Further, the image processor portion 120 transmits the generated display vertical synchronization signal to the scope distal portion 110.

Thereby, the imaging vertical synchronization signal and the display vertical synchronization signal are generated based on the master clock generated by the scope distal portion 110. Accordingly, the one-frame cycle for imaging can completely match that for displaying. Further, the TG 113 generates the imaging vertical synchronization signal so as to be synchronized with the display vertical synchronization signal received from the image processor portion 120. Then, the imaging device 114 captures an image at a timing based on the imaging vertical synchronization signal, and the image processing circuit 125 causes the monitor 140 to display the image at a timing based on the display vertical synchronization signal. As such, it is possible to secure the synchronization between the imaging and the displaying. The electronic endoscopic apparatus 1 can match the imaging cycle and the display cycle according to this structure.

Next, settings required to realize the operation of the present embodiment will be described.

### (Setting 1) Relation between transmission clock and reception clock

To completely match a frequency of the transmission clock and a frequency of the reception clock, the first multiplying/dividing circuit 112 generates the transmission clock, and the second multiplying/dividing circuit 121 generates the reception clock. The first multiplying/dividing circuit 112 generates the transmission clock from a master clock, and the second multiplying/dividing circuit 121 generates the reception clock from a transmission clock obtained by multiplying and/or dividing the master clock by (natural number/natural number). As such, the two clocks can be generated as clocks whose frequencies are completely identical to each other. Further, in terms of the reception clock, if the image signal transmitted from the scope distal portion 110 is a code conducting 8b/10b conversion, the reception clock can be recovered from the image signal by well-known technology.

### (Setting 2) Relation between imaging vertical synchronization signal and imaging clock

Since the number of pixels of the imaging device 114 is not always equal to that of the monitor 140, it is acceptable for the frequency of the imaging clock to be different from that of the display clock. However, the imaging vertical synchronization signal and the display vertical synchronization signal are only different in phase, and should be completely identical in cycle. Further, the frequency of the imaging clock is decided such that an edge timing of the imaging clock is always included in an edge timing of the imaging vertical synchronization signal. That is, the frequency of the imaging clock is decided such that the imaging device 114 can capture an image at a timing that is completely identical to that of the imaging vertical synchronization signal.

### (Setting 3) Relation between display vertical synchronization signal and display clock

A frequency of the display clock is decided such that an edge timing of the display clock is always included in an edge timing of the display vertical synchronization signal. That is, the frequency of the display clock is decided such that the image processing circuit 125 can display an image on the monitor 140 at a timing that is completely identical to that of the display vertical synchronization signal.

### (Setting 4) Method of determining frequency of imaging clock

For example, when the monitor 140 is a high definition television (HDTV), the cycle of the display vertical synchronization signal should be 59.94 Hz. Further, in this case, the display clock should have a frequency of 74.1758 MHz, or a frequency obtained by multiplying the frequency of 74.1758 MHz. On the other hand, since the cycle of the display vertical synchronization signal is 59.95 Hz, the cycle of the imaging vertical synchronization signal is also 59.95 Hz, but the frequency of the imaging clock is selected from various frequencies depending on the number of longitudinal and transverse pixels of the imaging device 114. Further, when the frequency of the imaging clock is selected, the frequency is required to be selected so that the imaging vertical synchronizasion signal can be generated by multiplying the frequency of the imaging clock, and the dispay clock can be generated by multiplying and/or dividing the frequency of the imaging clock by the integer. Further, the frequency of the imaging clock should select a frequency that becomes a multiple of an integer by the number of vertical lines of the imaging device is multiplied, even including an invalid period.

### (Setting 5) Method of determining transmission clock frequency

Recently, the number of pixels of the imaging device 114 is increasing, and at the same time, the frequency of the imaging clock is also increasing. In contrast, the transmission clock should be confined within a frequency capable of being transmitted by the long scope cable portion 130. For the transmission clock, the imaging clock is multiplied or divided by an integer and a frequency available for transmission that is lower than that of the imaging clock is selected. However, in this case, a frequency that is an integer times that of the imaging vertical synchronization signal and the display vertical synchronization signal is selected. Further, a display clock having precision required for displaying on the monitor should be generated from the transmission clock. Consequently, for example, when the monitor 140 is an HDTV, a frequency that can be generated by multiplying and/or dividing 74.1758 MHz, which is the frequency of the display clock, by the integer, should be adopted as the frequency of the transmission clock. Further, it is necessary to select the clock oscillator 111 that can generate a master clock having a frequency that can meet conditions of these settings.

### (Setting 6) Relation between imaging vertical synchronization signal and display vertical synchronization signal

The imaging device 114 is driven based on the imaging vertical synchronization signal. However, a slight delay takes place until the image signal is output from the imaging device 114. Further, even in the transmission of the scope cable portion 130, a delay occurs. In addition, even after the image signal arrives at the image processor portion 120 from the scope distal portion 110, a delay occurs due to processing inside of the image processor portion 120. For example, until a Bayer pattern image is interpolated to undergo three-pattern color conversion, conversion in a luminance pattern and a color difference pattern, filtering and zooming, and so on, and is converted into a pattern in which the image can be displayed on the monitor 140, a delay occurs. Accordingly, the imaging vertical synchronization signal is generated using a phase that is ahead of that of the display vertical synchronization signal by at least the sum of the aforementioned delay times. Thereby, the image captured by the imaging device 114 can be displayed on the monitor 140 at a fastest timing. Further, a capacity of a memory required to temporarily store the image signal for timing adjustment can be kept to the minimum extent.

As described above, according to the present embodiment, the clock oscillator 111 of the scope distal portion 110 outputs the master clock. Further, the first multiplying/dividing circuit 112 multiplies and/or divides the master clock by (natural number/natural number), thereby generating the imaging clock and the transmission clock. The transmission clock is transmitted to the second multiplying/dividing circuit 121 of the image processor portion 120. Further, the TG 113 receives the display vertical synchronization signal from the image processor portion 120, and generates the imaging vertical synchronization signal synchronized with the display vertical synchronization signal from the imaging clock. In addition, the imaging device 114 is operated by the imaging clock, captures the one-frame image at a timing based on the imaging vertical synchronization signal, and outputs the image signal.

Meanwhile, the second multiplying/dividing circuit 121 of the image processor portion 120 multiplies and/or divides the transmission clock transmitted from the scope distal portion 110 by (natural number/natural number), thereby generating the display clock. Further, the SSG 122 generates the display vertical synchronization signal from the display clock. Also, the display vertical synchronization signal generated by the SSG 122 is transmitted to the TG 113 of the scope distal portion 110. In addition, the image processing circuit 125 displays the one-frame image on the monitor 140 at a timing based on the display vertical synchronization signal generated by the SSG 122.

Thereby, the imaging vertical synchronization signal and the display vertical synchronization signal are generated based on the master clock generated by the scope distal portion 110. Accordingly, the one-frame cycle for imaging can completely match that for displaying. Further, the imaging device 114 can capture the one-frame image at a timing based on the imaging vertical synchronization signal, and the image processing circuit 125 can display the one-frame image on the monitor 140 at a timing based on the display vertical synchronization signal. As such, the electronic endoscopic apparatus 1 can secure the synchronization between the imaging and the displaying.

Accordingly, even when the processing of the imaging device 114 is performed at a high speed, the electronic endoscopic apparatus 1 can secure the synchronization between the cycle in which the scope distal portion 110 captures the image and the cycle in which the image processor portion 120 displays the image on the monitor 140. Thereby, the electronic endoscopic apparatus 1 can also suppress a phenomenon called "passing" or "frame dropping." Here, to simplify the description, description of timing control in which typical image processing such as correction, color conversion, filtering or the like is performed on the data (RAW) of the imaging device is omitted.

Further, in the present embodiment, a CMOS sensor is adopted as the imaging device 114, and the imaging device 114, the first multiplying/dividing circuit 112, the TG 113, the data transmission circuit 115, and the clock oscillator 111 other than a crystal oscillator are provided on the same chip, so that the number of components mounted on the scope distal portion 110 can be kept equivalent to that of an existing electronic endoscopic apparatus. Thus, as described above, the electronic endoscopic apparatus 1 of the present embodiment can suppress the frequency of the transmission clock, and suppress the occurrence of electromagnetic noise.

The electronic endoscopic apparatus 1 of the present embodiment can be subjected to various modifications. For example, the present embodiment is configured so that the display vertical synchronization signal is independently transmitted from the image processor portion 120 to the scope distal portion 110, but it is not limited to this configuration. Thus, the display vertical synchronization signal may be transmitted through a power line as a superimposed signal. Further, the present embodiment is configured so that the transmission clock is independently transmitted from the scope distal portion 110 to the image processor portion 120, but it is not limited to this configuration. Thus, the transmission clock may be superimposed on the image signal, and transmitted. In addition, the CMOS sensor is used as the imaging device 114, but the present embodiment is not limited to this configuration. Thus, a charge-coupled device (CCD) may be used. Further, the scope distal portion 110 and the image processor portion 120 are structurally independent of each other, but the present embodiment is not limited to this configuration. Thus, the scope distal portion 110 and the image processor portion 120 may have a monolithic structure.

### (Second Embodiment)

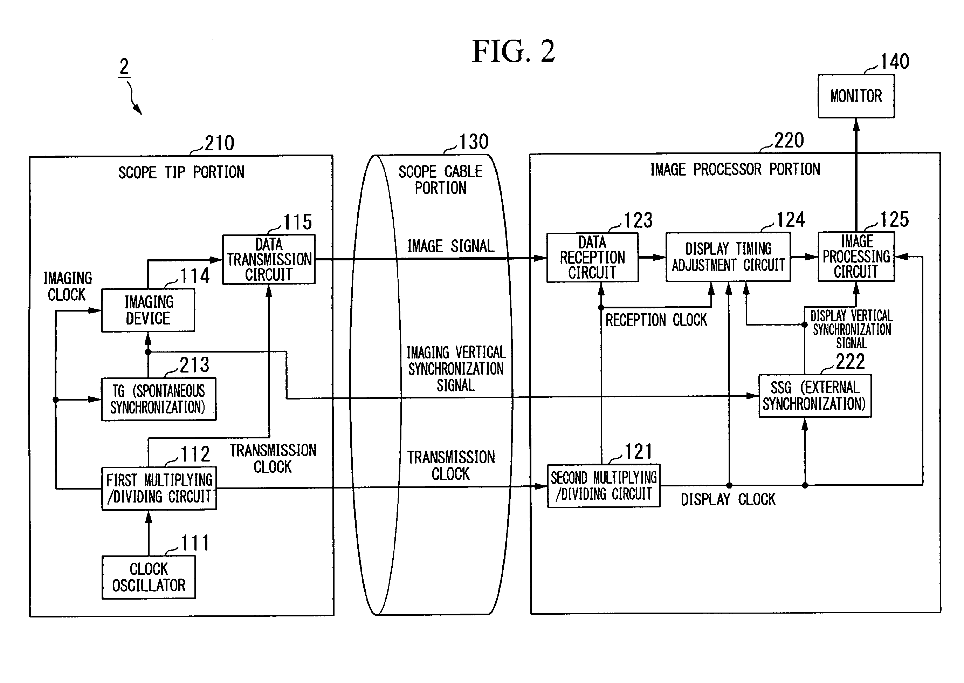

Next, a second embodiment of the present invention will be described. The second embodiment will be described focusing on differences from the first embodiment, and thus description of the same portions as the first embodiment will be omitted. FIG. 2 is a block diagram showing a configuration of an electronic endoscopic apparatus 2 in the present embodiment. In the shown example, the electronic endoscopic apparatus 2 includes a scope distal portion 210, an image processor portion 220, a scope cable portion 130, and a monitor 140.

The scope distal portion 210 includes a clock oscillator 111, a first multiplying/dividing circuit 112, a TG 213, an imaging device 114, and a data transmission circuit 115. The image processor portion 220 includes a second multiplying/dividing circuit 121, an SSG 222, a data reception circuit 123, a display timing adjustment circuit 124, and an image processing circuit 125.

Differences between the electronic endoscopic apparatus 2 of the present embodiment and the electronic endoscopic apparatus 1 of the first embodiment are only a configuration of the TG 213 of the scope distal portion 210, a configuration of the SSG 222 of the image processor portion 220, and a transmitting direction of the vertical synchronization signal within the scope cable portion 130. The other configurations are equivalent to the configurations of the components in the first embodiment.

The TG 213 generates various control signals from an imaging clock input by the first multiplying/dividing circuit 112, including an imaging vertical synchronization signal for driving the imaging device 114. The TG 213 in the present embodiment decides a start timing by itself and generates the imaging vertical synchronization signal without receiving input of the display vertical synchronization signal from the outside. Further, the display vertical synchronization signal generated by the TG 213 is transmitted to the image processor portion 220 via the scope cable portion 130. The imaging vertical synchronization signal transmitted to the image processor portion 220 is input to the SSG 222.

The imaging vertical synchronization signal transmitted from the scope distal portion 210 is input to the SSG 222. Further, the SSG 222 generates various control signals, which include a display vertical synchronization signal indicating a timing at which an image based on an image signal is displayed on the monitor 140, from the display clock generated by the second multiplying/dividing circuit 121, in a cycle that is completely identical to that of the input imaging vertical synchronization signal at a timing that is delayed more than a timing at which the display vertical synchronization signal is generated by at least a time that is the sum of a transmission delay time of the scope cable portion 130 and a processing time at the image processor portion 120. Thereby, in consideration of the delay, it is possible to secure synchronization between imaging and displaying. Unlike the first embodiment, the display vertical synchronization signal generated by the SSG 222 is not transmitted to the scope distal portion 210.

Next, an operation of the electronic endoscopic apparatus 2 of the present embodiment will be described. In the present embodiment, the scope distal portion 210 generates a master clock, multiplies and/or divides the master clock by (natural number/natural number), and generates an imaging clock and a transmission clock. Further, the scope distal portion 210 generates the imaging vertical synchronization signal from the imaging clock, and captures a one-frame image at a timing based on the imaging vertical synchronization signal. In addition, the scope distal portion 210 transmits the generated transmission clock and imaging vertical synchronization signal to the image processor portion 220.

On the other hand, the image processor portion 220 generates the display vertical synchronization signal from the transmitted transmission clock so as to be synchronized with the imaging vertical synchronization signal transmitted from the scope distal portion 210, and displays the one-frame image at a timing based on the display vertical synchronization signal.

Thereby, the imaging vertical synchronization signal and the display vertical synchronization signal are generated based on the master clock generated by the scope distal portion 210. Accordingly, the one-frame cycle for imaging can completely match that for displaying. Further, the SSG 222 generates the display vertical synchronization signal so as to be synchronized with the imaging vertical synchronization signal received from the scope distal portion 210. Then, the imaging device 114 captures an image at a timing based on the imaging vertical synchronization signal, and the image processing circuit 125 displays the image on the monitor 140 at a timing based on the display vertical synchronization signal. As such, it is possible to secure the synchronization between the imaging and the displaying. The electronic endoscopic apparatus 2 can match the imaging cycle and the display cycle according to this structure.

Next, settings required to realize the operation of the present embodiment will be described.
Settings 1 to 5 are the same as those in the first embodiment.

### (Setting 6) Relation between imaging vertical synchronization signal and display vertical synchronization signal

The imaging device 114 is driven based on the imaging vertical synchronization signal. However, a slight delay takes place until the image signal is output from the imaging device 114. Further, even in the transmission of the scope cable portion 130, a delay occurs. In addition, even after the image signal arrives at the image processor portion 220 from the scope distal portion 210, a delay occurs due to processing within the image processor portion 220. For example, until a Bayer pattern image is interpolated to undergo three-pattern color conversion, conversion in a luminance pattern and a color difference pattern, filtering and zooming, and so on, and is converted into a pattern in which the image can be displayed on the monitor 140, a delay occurs. Accordingly, the display vertical synchronization signal is generated using a phase that is delayed more than that of the imaging vertical synchronization signal by at least the sum of the aforementioned delay times. Thereby, the image captured by the imaging device 114 can be displayed on the monitor 140 at a fastest timing. Further, a capacity of a memory required to temporarily store the image signal for timing adjustment can be kept to the minimum extent.

As described above, according to the present embodiment, the clock oscillator 111 of the scope distal portion 210 outputs a master clock. Further, the first multiplying/dividing circuit 112 multiplies and/or divides the master clock by (natural number/natural number), thereby generating the imaging clock and the transmission clock. Further, the transmission clock is transmitted to the second multiplying/dividing circuit 121 of the image processor portion 220. Also, the TG 213 generates the imaging vertical synchronization signal from the imaging clock. Further, the imaging vertical synchronization signal is transmitted to the SSG 222 of the image processor portion 220. In addition, the imaging device 114 is operated by the imaging clock, captures the one-frame image at a timing based on the imaging vertical synchronization signal, and outputs the image signal.

On the other hand, the second multiplying/dividing circuit 121 of the image processor portion 220 multiplies and/or divides the transmission clock transmitted from the scope distal portion 210 by (natural number/natural number), thereby generating the display clock. Further, the SSG 222 receives the imaging vertical synchronization signal from the scope distal portion 210, and generates the display vertical synchronization signal synchronized with the imaging vertical synchronization signal from the display clock.
In addition, the image processing circuit 125 displays the one-frame image on the monitor 140 at a timing based on the display vertical synchronization signal generated by the SSG 222.

Thereby, the imaging vertical synchronization signal and the display vertical synchronization signal are generated based on the master clock generated by the scope distal portion 210. Accordingly, the one-frame cycle for imaging can completely match that for displaying. Further, the imaging device 114 can capture the one-frame image at a timing based on the imaging vertical synchronization signal, and the image processing circuit 125 can display the one-frame image on the monitor 140 at a timing based on the display vertical synchronization signal. As such, the electronic endoscopic apparatus 2 can secure the synchronization between the imaging and the displaying.

Accordingly, even when the processing of the imaging device 114 is performed at a high speed, the electronic endoscopic apparatus 2 can secure the synchronization between the cycle in which the scope distal portion 210 captures the image and the cycle in which the image processor portion 220 displays the image on the monitor 140. Thereby, the electronic endoscopic apparatus 2 can also suppress a phenomenon called "passing" or "frame dropping." Here, to simplify the description, description of timing control that performs typical image processing such as correction, color conversion, filtering or the like on the data (RAW) of the imaging device is omitted.

Further, in the present embodiment, a CMOS sensor is adopted as the imaging device 114, and the imaging device 114, the first multiplying/dividing circuit 112, the TG 213, the data transmission circuit 115, and the clock oscillator 111 other than a crystal oscillator are provided on the same chip, so that the number of components mounted on the scope distal portion 210 can be kept equivalent to that of an existing electronic endoscopic apparatus. Thus, as described above, the electronic endoscopic apparatus 2 of the present embodiment can suppress the frequency of the transmission clock, and suppress the occurrence of electromagnetic noise.

The electronic endoscopic apparatus 2 of the present embodiment can be subjected to various modifications. For example, the present embodiment is configured so that the imaging vertical synchronization signal and the transmission clock are independently transmitted from the scope distal portion 210 to the image processor portion 220, but it is not limited to this configuration. Thus, the imaging vertical synchronization signal and the transmission clock may be superimposed on the image signal and transmitted. Further, the CMOS sensor is used as the imaging device 114, but the present embodiment is not limited to this configuration. Thus, a CCD may be used. Further, the scope distal portion 210 and the image processor portion 220 are structurally independent of each other, but the present embodiment is not limited to this configuration. Thus, the scope distal portion 210 and the image processor portion 220 may have a monolithic structure.

### (Third Embodiment)

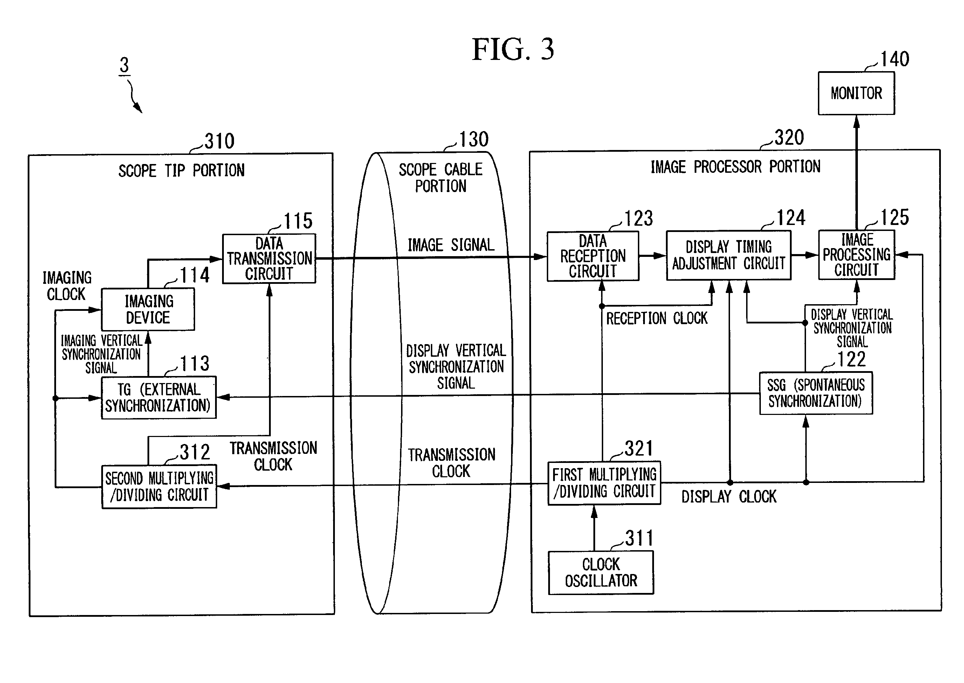

Next, a third embodiment of the present invention will be described. The third embodiment will be described focusing on differences from the first embodiment, and thus description of the same portions as the first embodiment will be omitted. FIG. 3 is a block diagram showing a configuration of an electronic endoscopic apparatus 3 in the present embodiment. In the shown example, the electronic endoscopic apparatus 3 includes a scope distal portion 310, an image processor portion 320, a scope cable portion 130, and a monitor 140.

The scope distal portion 310 includes a second multiplying/dividing circuit 312, a TG 113, an imaging device 114, and a data transmission circuit 115. The image processor portion 320 includes a clock oscillator 311, a first multiplying/dividing circuit 321, an SSG 122, a data reception circuit 123, a display timing adjustment circuit 124, and an image processing circuit 125.

The electronic endoscopic apparatus 3 in the present embodiment is different from the electronic endoscopic apparatus 1 in the first embodiment in that the scope distal portion 310 has the second multiplying/dividing circuit 312 in place of the first multiplying/dividing circuit 112 without the clock oscillator, and in that the image processor portion 320 has the clock oscillator 311 and the first multiplying/dividing circuit 321 in place of the second multiplying/dividing circuit 121. The other configurations are equal to the configurations of the components in the first embodiment.

The clock oscillator 311 of the image processor portion 320 is an oscillator such as a crystal oscillator, and generates a master clock. The first multiplying/dividing circuit 321 multiplies and/or divides the master clock by (natural number/natural number), generates a reception clock having a frequency required to receive the image signal transmitted from the scope distal portion 310, and outputs the generated reception clock to the data reception circuit 123 and the display timing adjustment circuit 124. Further, the first multiplying/dividing circuit 321 multiplies and/or divides the master clock by (natural number/natural number), generates a display clock having a frequency required to display an image, which is based on the image signal transmitted from the scope distal portion 310, on the monitor 140, and outputs the generated display clock to the SSG 122, the display timing adjustment circuit 124, and the image processing circuit 125. In addition, the first multiplying/dividing circuit 321 generates a transmission clock to be transmitted to the scope distal portion 310. The transmission clock generated by the first multiplying/dividing circuit 321 is transmitted to the scope distal portion 310 via the scope cable portion 130.

The second multiplying/dividing circuit 312 of the scope distal portion 310 receives the transmission clock transmitted from the image processor portion 320. The second multiplying/dividing circuit 312 multiplies and/or divides the received transmission clock by (natural number/natural number), and generates an imaging clock for driving the imaging device 114 and the TG 113 and a transmission clock to be fed to the data transmission circuit 115.

Next, an operation of the electronic endoscopic apparatus 3 in the present embodiment will be described. In the present embodiment, the image processor portion 320 generates a master clock, multiplies and/or divides the master clock by (natural number/natural number), and generates a display clock and a transmission clock. Further, the image processor portion 320 generates a display vertical synchronization signal from a display clock, and displays a one-frame image on the monitor 140 at a timing based on the display vertical synchronization signal. In addition, the image processor portion 320 transmits the generated transmission clock and display vertical synchronization signal to the image processor portion 320.

On the other hand, the scope distal portion 310 generates an imaging vertical synchronization signal from the transmitted transmission clock so as to be synchronized with the display vertical synchronization signal transmitted from the image processor portion 320, and displays a one-frame image at a timing based on the imaging vertical synchronization signal.

Thereby, the imaging vertical synchronization signal and the display vertical synchronization signal are generated based on the master clock generated by the image processor portion 320. Accordingly, the one-frame cycle for imaging can completely match that for displaying. Further, the TG 113 generates the imaging vertical synchronization signal so as to be synchronized with the display vertical synchronization signal received from the image processor portion 320. Then, the imaging device 114 captures an image at a timing based on the imaging vertical synchronization signal, and the image processing circuit 125 causes the monitor 140 to display the image at a timing based on the display vertical synchronization signal. As such, it is possible to secure the synchronization between the imaging and the displaying. The electronic endoscopic apparatus 3 can match the imaging cycle and the display cycle according to this structure.

Next, settings required to realize the operation of the present embodiment will be described. Settings 1 to 6 in the present embodiment are the same as those in the first embodiment.
The present embodiment is different from the first embodiment in that the transmission clock is generated by multiplying and/or dividing the display clock at the first multiplying/dividing circuit 321 of the image processor portion 320, and is transmitted to the scope distal portion 310.

As described above, according to the present embodiment, the second multiplying/dividing circuit 312 of the scope distal portion 310 multiplies and/or divides the transmission clock transmitted from the image processor portion 320 by (natural number/natural number), thereby generating the imaging clock. Further, the TG 113 receives the display vertical synchronization signal from the image processor portion 320, and generates the imaging vertical synchronization signal synchronized with the display vertical synchronization signal from the imaging clock. In addition, the imaging device 114 is operated by the imaging clock, captures the one-frame image at a timing based on the imaging vertical synchronization signal, and outputs image data.

Meanwhile, the clock oscillator 311 of the image processor portion 320 outputs a master clock.
Further, the first multiplying/dividing circuit 321 multiplies and/or divides the master clock by (natural number/natural number), thereby generating a display clock and a transmission clock. Further, the transmission clock is transmitted to the second multiplying/dividing circuit 312 of the scope distal portion 310. Also, the SSG 122 generates a display vertical synchronization signal from the display clock. Further, the display vertical synchronization signal generated by the SSG 122 is transmitted to the TG 113 of the scope distal portion 310. In addition, the image processing circuit 125 displays a one-frame image on the monitor 140 at a timing based on the display vertical synchronization signal generated by the SSG 122.

Thereby, the imaging vertical synchronization signal and the display vertical synchronization signal are generated based on the master clock generated by the image processor portion 320. Accordingly, the one-frame cycle for imaging can completely match that for displaying. Further, the imaging device 114 can capture the one-frame image at a timing based on the imaging vertical synchronization signal, and the image processing circuit 125 can display the one-frame image on the monitor 140 at a timing based on the display vertical synchronization signal. As such, the electronic endoscopic apparatus 3 can secure the synchronization between the imaging and the displaying.

Accordingly, even when the processing of the imaging device 114 is performed at a high speed, the electronic endoscopic apparatus 3 can secure the synchronization between the cycle in which the scope distal portion 310 captures the image and the cycle in which the image processor portion 320 displays the image on the monitor 140. Thereby, the electronic endoscopic apparatus 3 can also suppress a phenomenon called "passing" or "frame dropping." Here, to simplify the description, description of timing control in which typical image processing such as correction, color conversion, filtering or the like is performed on the data (RAW) of the imaging device is omitted.

Further, in the present embodiment, a CMOS sensor is adopted as the imaging device 114, and the imaging device 114, the second multiplying/dividing circuit 312, the TG 113, and the data transmission circuit 115 are provided on the same chip, so that the number of components mounted on the scope distal portion 310 can be kept equivalent to that of an existing electronic endoscopic apparatus. Thus, as described above, the electronic endoscopic apparatus 3 of the present embodiment can suppress the frequency of the transmission clock, and suppress the occurrence of electromagnetic noise.

The electronic endoscopic apparatus 3 of the present embodiment can be subjected to various modifications. For example, the present embodiment is configured so that the display vertical synchronization signal and the transmission clock are independently transmitted from the image processor portion 320 to the scope distal portion 310, but it is not limited to this configuration. Thus, the display vertical synchronization signal and the transmission clock may be transmitted through a power line as a superimposed signal. Further, the CMOS sensor is used as the imaging device 114, but the present embodiment is not limited to this configuration. Thus, a CCD may be used. Further, the scope distal portion 310 and the image processor portion 320 are structurally independent of each other, but the present embodiment is not limited to this configuration. Thus, the scope distal portion 310 and the image processor portion 320 may have a monolithic structure.

### (Fourth Embodiment)

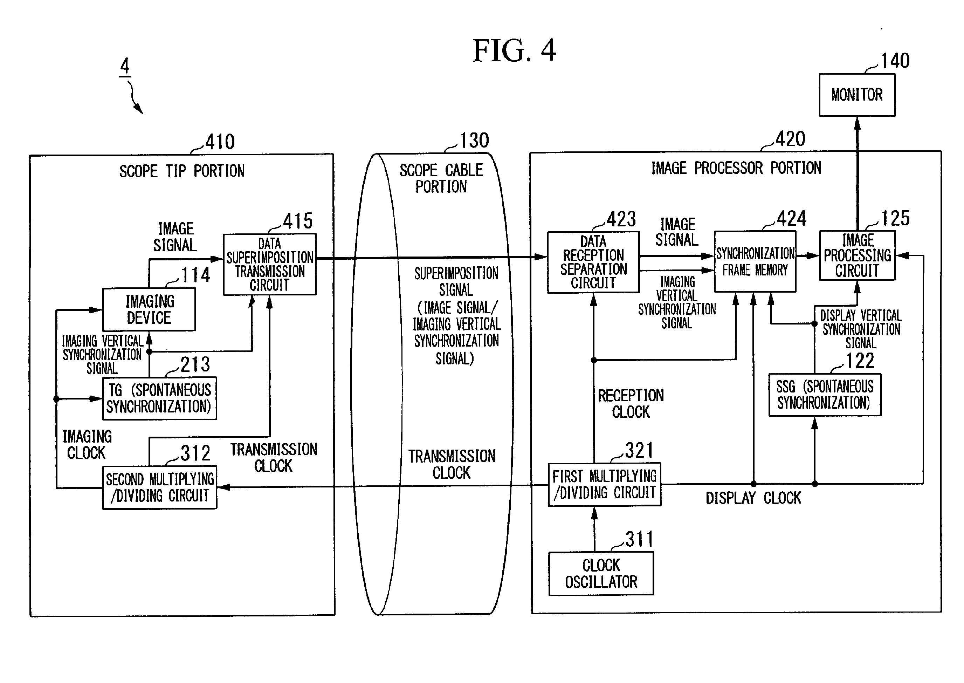

Next, a fourth embodiment of the present invention will be described. The fourth embodiment will be described focusing on differences from the first embodiment, and thus description of the same portions as the first embodiment will be omitted. FIG. 4 is a block diagram showing a configuration of an electronic endoscopic apparatus 4 in the present embodiment. In the shown example, the electronic endoscopic apparatus 4 includes a scope distal portion 410, an image processor portion 420, a scope cable portion 130, and a monitor 140.

The scope distal portion 410 includes a second multiplying/dividing circuit 312, a TG 213, an imaging device 114, and a data superimposition transmission circuit 415. The image processor portion 420 includes a clock oscillator 311, a first multiplying/dividing circuit 321, an SSG 122, a data reception separation circuit 423, a synchronization frame memory 424, and an image processing circuit 125.

The electronic endoscopic apparatus 4 in the present embodiment is different from the electronic endoscopic apparatus 1 in the first embodiment in that the scope distal portion 410 does not include the clock oscillator and has the second multiplying/dividing circuit 312 in place of the first multiplying/dividing circuit 112 and the data superimposition transmission circuit 415 in place of the data transmission circuit 115, in that the image processor portion 420 includes the clock oscillator 311 and has the first multiplying/dividing circuit 321 in place of the second multiplying/dividing circuit 121, the data reception separation circuit 423 in place of the data reception circuit 123, and the synchronization frame memory 424 in place of the display timing adjustment circuit 124, and in that the vertical synchronization signal is transmitted within the scope cable portion 130 in the reverse direction and is superimposed on and transmitted with the image signal. The other configurations are equivalent to the configurations of the components in the first embodiment.

The clock oscillator 311 of the image processor portion 420 is an oscillator such as a crystal oscillator, and generates a master clock. The first multiplying/dividing circuit 321 multiplies and/or divides the master clock by (natural number/natural number), generates a reception clock having a frequency required to receive the image signal transmitted from the scope distal portion 310, and outputs the generated reception clock to the data reception separation circuit 423 and the synchronization frame memory 424. Further, the first multiplying/dividing circuit 321 multiplies and/or divides the master clock by (natural number/natural number), generates a display clock having a frequency required to display an image, which is based on the image signal transmitted from the scope distal portion 410, on the monitor 140, and outputs the generated display clock to the SSG 122, the synchronization frame memory 424, and the image processing circuit 125. In addition, the first multiplying/dividing circuit 321 generates a transmission clock to be transmitted to the scope distal portion 410. The transmission clock generated by the first multiplying/dividing circuit 321 is transmitted to the scope distal portion 410 via the scope cable portion 130.

The SSG 122 generates a display vertical synchronization signal indicating a timing at which the image based on the image signal is displayed on the monitor 140, and a variety of control signals from the display clock input from the first multiplying/dividing circuit 321, and outputs the generated signals to the synchronization frame memory 424 and the image processing circuit 125. Note that the display vertical synchronization signal generated by the SSG 122 is not transmitted to the scope distal portion 410.

After the data reception separation circuit 423 receives a superimposition signal, and converts the superimposition signal from a differential pattern to a typical pattern (single-end), the data reception separation circuit 423 determines a specific pattern of synchronization signal and separates the specific pattern as an imaging vertical synchronization signal. Simultaneously, the data reception separation circuit 423 distinguishes a head of the image signal to conduct reverse conversion of 8b/10b and conversion from a serial pattern to a parallel pattern, and writes the image signal after the conversion from a first port of the synchronization frame memory using the reception clock. The synchronization frame memory 424 stores the image signal.
The image processing circuit 125 reads out the image signal from a second port of the synchronization frame memory 424 at a timing based on the display vertical synchronization signal generated by the SSG 122 using the display clock. Then, the image processing circuit 125 performs image processing on the read image signal, and displays the image (one-frame image) based on the image signal on the monitor 140 at a timing based on the display vertical synchronization signal generated by the SSG 112.

The second multiplying/dividing circuit 312 of the scope distal portion 410 receives the transmission clock transmitted from the image processor portion 420. Then, the second multiplying/dividing circuit 312 multiplies and/or divides the received transmission clock by (natural number/natural number), and generates an imaging clock for driving the imaging device 114 and the TG 213 and a transmission clock to be fed to the data superimposition transmission circuit 415. The TG 213 generates a variety of control signals including an imaging vertical synchronization signal for driving the imaging device 114 from the imaging clock input from the second multiplying/dividing circuit 312. In the present embodiment, the TG 213 decides a start timing by itself and generates the imaging vertical synchronization signal, without receiving input of the display vertical synchronization signal from the outside.

The data superimposition transmission circuit 415 superimposes the imaging vertical synchronization signal generated by the TG 213 on the image signal output by the imaging device 114, thereby generating the superimposition signal. Further, the data superimposition transmission circuit 415 converts the superimposition signal into a pattern in which the superimposition signal can be transmitted to the image processor portion 420, and transmits the converted superimposition signal to the image processor portion 420 via the scope cable portion 130. As a method of superimposing the imaging vertical synchronization signal and converting the superimposed signal in a pattern available for transmission, for example, the following method may be used. That is, the image signal, which is output by the imaging device 114 and has a pattern in which a pixel value is expressed in a multi-bit parallel form, into a serial form, and conducting 8b/10b conversion. On the other hand, the imaging vertical synchronization signal is converted into a specific code pattern that is not expressed after the 8b/10b conversion, substituting the converted signal with data of an invalid period corresponding to a vertical flyback period of the imaging signal, and converting the substituted data into a differential pattern.

Next, an operation of the electronic endoscopic apparatus 4 in the present embodiment will be described. In the present embodiment, the image processor portion 420 generates a master clock, multiplies and/or divides the master clock by (natural number/natural number), and generates a display clock and a transmission clock. Further, the image processor portion 420 generates a display vertical synchronization signal from the display clock, and displays a one-frame image on the monitor 140 at a timing based on the display vertical synchronization signal. Also, the image processor portion 420 transmits the generated transmission clock to the image processor portion 420. On the other hand, the scope distal portion 410 generates an imaging vertical synchronization signal from the transmitted transmission clock, and displays a one-frame image at a timing based on the imaging vertical synchronization signal.

Thereby, the imaging vertical synchronization signal and the display vertical synchronization signal are generated based on the master clock generated by the image processor portion 420. Accordingly, the one-frame cycle for imaging can completely match that for displaying. Further, the TG 213 and the SSG 122 generate the imaging vertical synchronization signal and the display vertical synchronization signal such that the cycles thereof are identical to each other. Then, the imaging device 114 captures an image at a timing based on the imaging vertical synchronization signal, and the image processing circuit 125 causes the monitor 140 to display the image at a timing based on the display vertical synchronization signal. As such, it is possible to secure the synchronization between the imaging and the displaying. The electronic endoscopic apparatus 4 can match the imaging cycle and the display cycle according to this structure. In the present embodiment, the start timing of the imaging is not always identical to that of the displaying. However, the synchronization frame memory temporarily storing the image signal, thereby a shift between these timings can be absorbed.

Next, settings required to realize the operation of the present embodiment will be described. Settings 1, 3, 4 and 5 are the same as those in the first embodiment.

### (Setting 2) Relation between imaging vertical synchronization signal and imaging clock

In the present embodiment, the image processor portion 420 has the synchronization frame memory 424. For this reason, if display passing (frame overlapping display or frame dropping display) caused by a difference between frame rates of the imaging and the displaying is permitted, the image based on the image signal can be displayed on the monitor 140 even when the present setting is not present. To prevent the display passing, the same setting as the first embodiment is required.

### (Setting 6) Relation between imaging vertical synchronization signal and display vertical synchronization signal

In the present embodiment, since the timing of the imaging side and the timing of the display side are switched by the synchronization frame memory 424, Setting 6 is not required. However, to prevent the display passing caused by the difference between the frame rates of the imaging and the displaying, the imaging vertical synchronization signal and the display vertical synchronization signal are preferably set so as to have the same cycle.

As described above, according to the present embodiment, the second multiplying/dividing circuit 312 of the scope distal portion 410 multiplies and/or divides the transmission clock transmitted from the image processor portion 420 by (natural number/natural number), thereby generating the imaging clock. Further, the TG 213 generates the imaging vertical synchronization signal from the imaging clock. In addition, the imaging device 114 is operated by the imaging clock, captures the one-frame image at a timing based on the imaging vertical synchronization signal, and outputs image data.

Meanwhile, the clock oscillator 311 of the image processor portion 420 outputs a master clock.
Further, the first multiplying/dividing circuit 321 multiplies and/or divides the master clock by (natural number/natural number), thereby generating a display clock and a transmission clock. Further, the transmission clock is transmitted to the second multiplying/dividing circuit 312 of the scope distal portion 410. Also, the SSG 122 generates a display vertical synchronization signal from the display clock. Further, the image processing circuit 125 causese the monitor 140 to display a one-frame image at a timing based on the display vertical synchronization signal generated by the SSG 122.

Thereby, the imaging vertical synchronization signal and the display vertical synchronization signal are generated based on the master clock generated by the image processor portion 420. Accordingly, the one-frame cycle for imaging can completely match that for displaying. Further, the imaging device 114 can capture the one-frame image at a timing based on the imaging vertical synchronization signal, and the image processing circuit 125 can cause the monitor 140 to display the one-frame image at a timing based on the display vertical synchronization signal. As such, by matching the cycle of the imaging vertical synchronization signal and the cycle of the display vertical synchronization signal, the electronic endoscopic apparatus 4 can secure the synchronization between the imaging and the displaying.

Accordingly, even when the processing of the imaging device 1 14 is performed at a high speed, the electronic endoscopic apparatus 4 can secure the synchronization between the cycle in which the scope distal portion 410 captures the image and the cycle in which the image processor portion 420 displays the image on the monitor 140. Thereby, the electronic endoscopic apparatus 4 can also suppress a phenomenon called "passing" or "frame dropping." Here, to simplify the description, description of timing control in which typical image processing such as correction, color conversion, filtering or the like is performed on the data (RAW) of the imaging device is omitted.

In the present embodiment, even when the imaging vertical synchronization signal and the display vertical synchronization signal are set to the same cycle, a structure in which the phase is maintained in a constant relation is not provided. Accordingly, a delay time from when the image is captured to when the image is displayed is altered depending on an application timing of power or an exchange timing of the scope. However, since the electronic endoscopic apparatus 4 continuously output the display vertical synchronization signal, the synchronization between when the image is captured and when the image is displayed on the monitor 140 is not disturbed even when the scope distal portion 410 is exchanged during operation.

Further, in the present embodiment, a CMOS sensor is adopted as the imaging device 114, and the imaging device 114, the second multiplying/dividing circuit 312, the TG 213, and the data superimposition transmission circuit 415 are provided on the same chip, so that the number of components mounted on the scope distal portion 410 can be kept equivalent to that of an existing electronic endoscopic apparatus. Thus, as described above, the electronic endoscopic apparatus 4 of the present embodiment can suppress the frequency of the transmission clock, and suppress the occurrence of electromagnetic noise.

Further, the present embodiment can be subjected to various modifications. For example, the present embodiment is configured so that the transmission clock is independently transmitted from the image processor portion 420 to the scope distal portion 410. However, the transmission clock may be transmitted from the image processor portion 420 to the scope distal portion 410 through a power line as a superimposed signal. Further, imaging vertical synchronization signal is configured to be superimposed on the image signal and transmitted from the scope distal portion 410 to the image processor portion 420. However, the vertical synchronization signal and the image signal may be independently transmitted. Also, the CMOS sensor is used as the imaging device 114, but the present embodiment is not limited to this configuration. Thus, a CCD may be used. In addition, the scope distal portion 410 and the image processor portion 420 are structurally independent of each other, but the present embodiment is not limited to this configuration. Thus, the scope distal portion 410 and the image processor portion 420 may have a monolithic structure.

While the first to fourth embodiments of the present invention have been described in detail with reference to the drawings, specific configurations are not limited to these embodiments, and include design modifications within a scope not departing from the gist of the present invention.

For example, the SSG 122 of the image processor portion 420 in the fourth embodiment may be changed into the SSG 222 of the image processor portion 220 in the second embodiment.

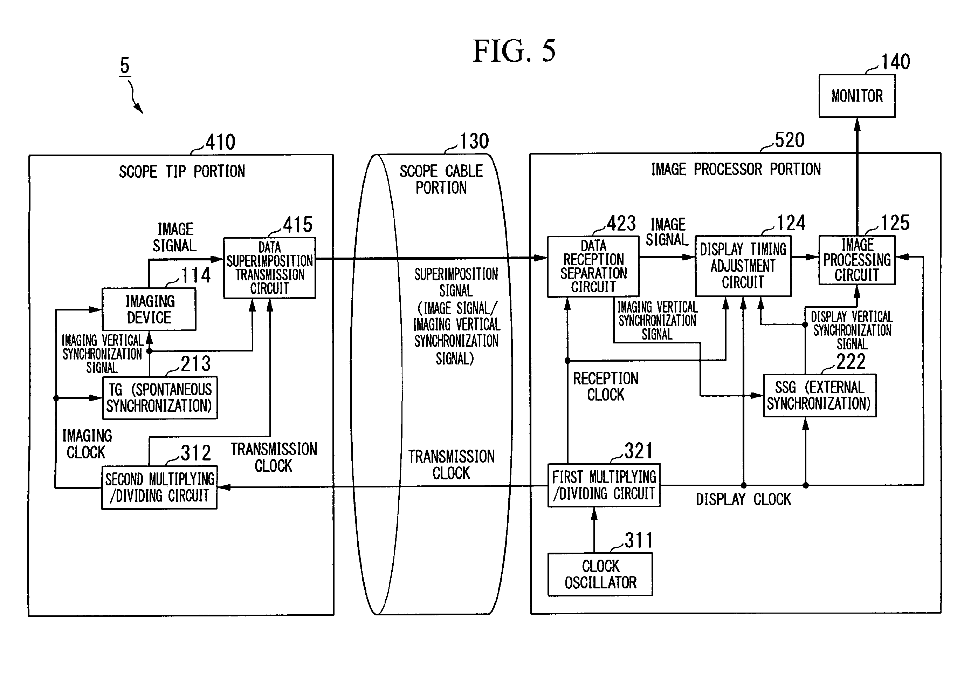

FIG. 5 is a block diagram showing a configuration of an electronic endoscopic apparatus 5 that is a modified example of the electronic endoscopic apparatus 4 in the fourth embodiment. In the shown example, the electronic endoscopic apparatus 5 includes a scope distal portion 410, an image processor portion 520, a scope cable portion 130, and a monitor 140. The image processor portion 520 includes a clock oscillator 311, a first multiplying/dividing circuit 321, an SSG 222, a data reception separation circuit 423, a display timing adjustment circuit 124, and an image processing circuit 125.

Configurations of the SSG 222 and the display timing adjustment circuit 124 are similar to those in the second embodiment. Further, an imaging vertical synchronization signal separated by the data reception separation circuit 423 is output to the SSG 222. The other configurations of the electronic endoscopic apparatus 5 are similar to the configurations of the electronic endoscopic apparatus 4.

In this case, the SSG 222 generates various control signals, which include a display vertical synchronization signal indicating a timing at which an image based on an image signal is displayed on the monitor 140, from a display clock generated by the first multiplying/dividing circuit 321, in a cycle that is completely identical to that of the imaging vertical synchronization signal input from the data reception separation circuit 423 at a timing that is delayed more than a timing at which the imaging vertical synchronization signal is generated by at least a time that is the sum of a transmission delay time of the scope cable portion 130 and a processing time at the image processor portion 520. Thereby, in consideration of the delay, it is possible to secure synchronization between imaging and displaying. Further, a synchronization frame memory 424 can thereby be changed into the small-scale display timing adjustment circuit 124 shown in the first embodiment, and can be configured to add the conditions of Setting 6.

As described above, according to this configuration, the second multiplying/dividing circuit 312 of the scope distal portion 410 multiplies and/or divides the transmission clock transmitted from the image processor portion 520 by (natural number/natural number), thereby generating an imaging clock. Further, the TG 213 generates an imaging vertical synchronization signal from the imaging clock. In addition, the imaging device 114 is operated by the imaging clock, captures a one-frame image at a timing based on the imaging vertical synchronization signal, and outputs image data.

On the other hand, the data reception separation circuit 423 separates the imaging vertical synchronization signal from the received superimposition signal, and outputs the separated imaging vertical synchronization signal to the SSG 222. Further, the clock oscillator 311 of the image processor portion 520 outputs a master clock. Also, the first multiplying/dividing circuit 321 multiplies and/or divides the master clock by (natural number/natural number), thereby generating a display clock and a transmission clock. Further, the transmission clock is transmitted to the second multiplying/dividing circuit 312 of the scope distal portion 410. Further, the SSG 222 generates various control signals, which include the display vertical synchronization signal indicating a timing at which the image based on the image signal is displayed on the monitor 140, from the display clock generated by the first multiplying/dividing circuit 321, in a cycle that is completely identical to that of the imaging vertical synchronization signal input from the data reception separation circuit 423 at a timing that is delayed more than a timing at which the imaging vertical synchronization signal is generated by at least a time that is the sum of a transmission delay time of the scope cable portion 130 and a processing time at the image processor portion 520. Thereby, in consideration of the delay, it is possible to secure synchronization between imaging and displaying. Further, the image processing circuit 125 causes the monitor 140 to display the one-frame image at a timing based on the display vertical synchronization signal generated by the SSG 222.

Thereby, the imaging vertical synchronization signal and the display vertical synchronization signal are generated based on the master clock generated by the image processor portion 520. Accordingly, the one-frame cycle for imaging can completely match that for displaying. Further, the imaging device 114 can capture the one-frame image at a timing based on the imaging vertical synchronization signal, and the image processing circuit 125 can causes the monitor 140 to display the one-frame imageat a timing based on the display vertical synchronization signal. As such, the electronic endoscopic apparatus 5 can secure the synchronization between the imaging and the displaying.

Accordingly, even when the processing of the imaging device 114 is performed at a high speed, the electronic endoscopic apparatus 5 can secure the synchronization between the cycle in which the scope distal portion 410 captures the image and the cycle in which the image processor portion 520 displays the image on the monitor 140. Thereby, the electronic endoscopic apparatus 5 can also suppress a phenomenon called "passing" or "frame dropping." Here, to simplify the description, description of timing control in which typical image processing such as correction, color conversion, filtering or the like is performed on the data (RAW) of the imaging device is omitted.

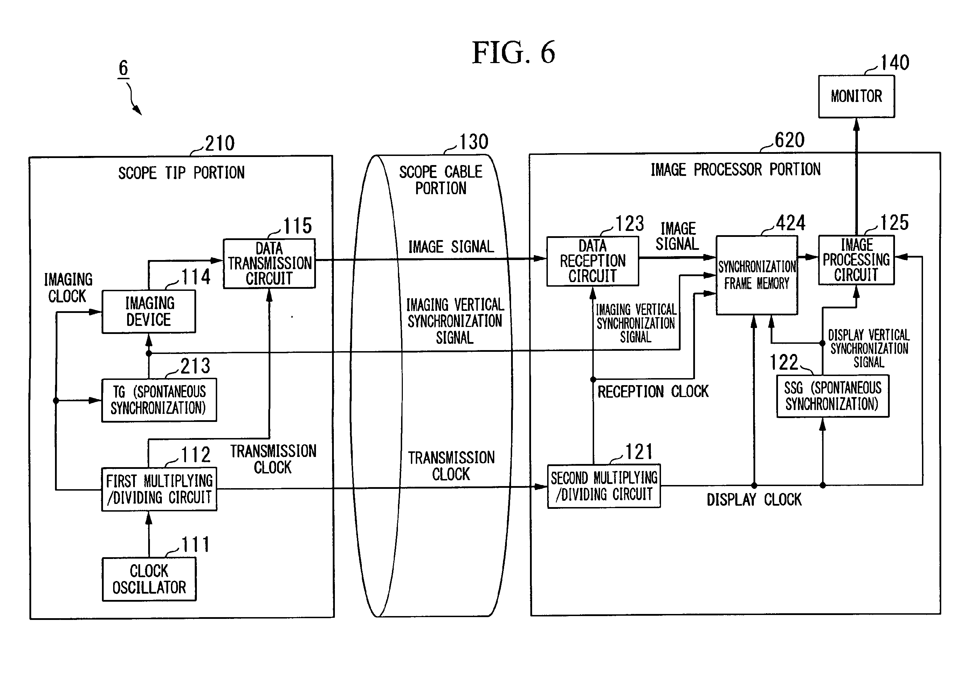

Further, similar to the image processor portion 420 in the fourth embodiment, the configuration of the image processor portion 220 in the second embodiment may include the synchronization frame memory 424 in place of the display timing adjustment circuit 124. FIG. 6 is a block diagram showing a configuration of an electronic endoscopic apparatus 6 that is a modified example of the electronic endoscopic apparatus 2 in the second embodiment. In the shown example, the electronic endoscopic apparatus 6 includes a scope distal portion 210, an image processor portion 620, a scope cable portion 130, and a monitor 140. The image processor portion 620 includes a second multiplying/dividing circuit 121, an SSG 122, a data reception circuit 123, a synchronization frame memory 424, and an image processing circuit 125.

Configurations of the synchronization frame memory 424 and the SSG 222 are similar to those in the fourth embodiment. Further, an imaging vertical synchronization signal transmitted from the scope distal portion 210 is input to the synchronization frame memory 424. The other configurations of the electronic endoscopic apparatus 6 are similar to the configurations of the electronic endoscopic apparatus 2.

With this configuration, the clock oscillator 111 of the scope distal portion 210 outputs a master clock. Further, the first multiplying/dividing circuit 112 multiplies and/or divides the master clock by (natural number/natural number), thereby generating an imaging clock and a transmission clock. Further, the transmission clock is transmitted to the second multiplying/dividing circuit 121 of the image processor portion 620. Also, the TG 213 generates the imaging vertical synchronization signal from the imaging clock. Further, the imaging vertical synchronization signal is transmitted to the synchronization frame memory 424 of the image processor portion 620. In addition, the imaging device 114 is operated by the imaging clock, captures a one-frame image at a timing based on the imaging vertical synchronization signal, and outputs image data.

On the other hand, the second multiplying/dividing circuit 121 of the image processor portion 620 multiplies and/or divides the transmission clock transmitted from the scope distal portion 210 by (natural number/natural number), thereby generating a display clock. Further, the SSG 122 generates a display vertical synchronization signal from the display clock. Also, the image processing circuit 125 causes the monitor 140 to display the one-frame image at a timing based on the display vertical synchronization signal generated by the SSG 122.

Thereby, the imaging vertical synchronization signal and the display vertical synchronization signal are generated based on the master clock generated by the scope distal portion 210. Accordingly, the one-frame cycle for imaging can completely match that for displaying. Further, the imaging device 114 can capture the one-frame image at a timing based on the imaging vertical synchronization signal, and the image processing circuit 125 can causes the monitor 140 to display the one-frame imageat a timing based on the display vertical synchronization signal. As such, the electronic endoscopic apparatus 6 can secure the synchronization between the imaging and the displaying.

Accordingly, even when the processing of the imaging device 1 14 is performed at a high speed, the electronic endoscopic apparatus 6 can secure the synchronization between the cycle in which the scope distal portion 210 captures the image and the cycle in which the image processor portion 620 displays the image on the monitor 140. Thereby, the electronic endoscopic apparatus 6 can also suppress a phenomenon called "passing" or "frame dropping." Here, to simplify the description, description of timing control in which typical image processing such as correction, color conversion, filtering or the like is performed on the data (RAW) of the imaging device is omitted.

In the present embodiment, even when the imaging vertical synchronization signal and the display vertical synchronization signal are set to the same cycle, a structure in which the phase is maintained in a constant relation is not provided. Accordingly, a delay time from when the image is captured to when the image is displayed is altered depending on an application timing of power or an exchange timing of the scope. However, since the electronic endoscopic apparatus 6 continuously outputs operation of the display vertical synchronization signal, the synchronization between when the image is captured and when the image is displayed on the monitor 140 is not disturbed even when the scope distal portion 210 is exchanged during operation.

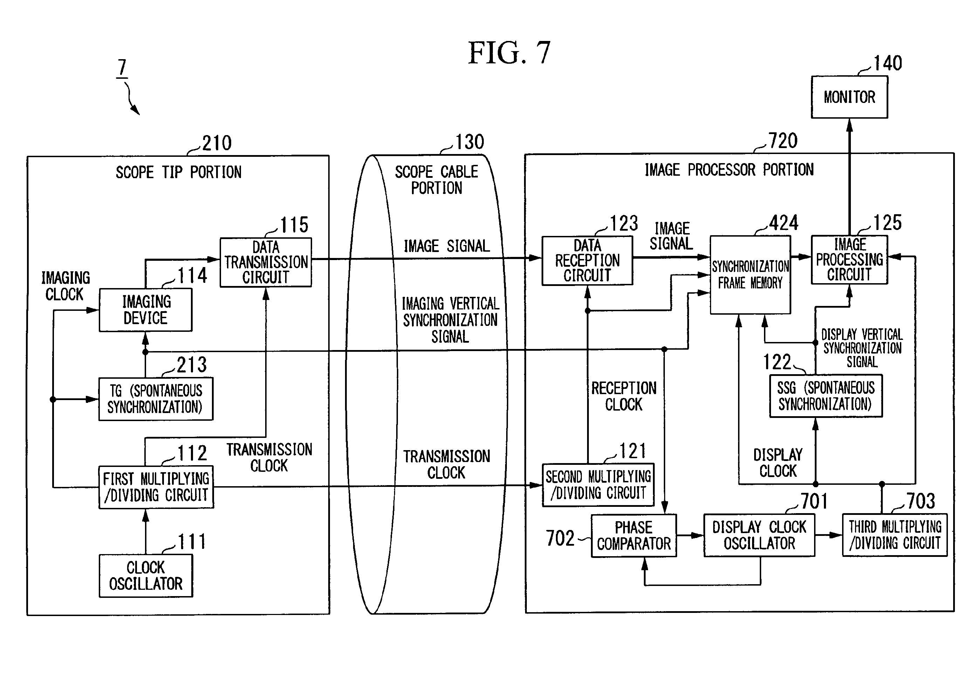

Further, the image processor portion 620 of the electronic endoscopic apparatus 6 shown in FIG. 6 may be configured to include a phase comparator, a display clock oscillator, and a third multiplying/dividing circuit. FIG. 7 is a block diagram showing a configuration of an electronic endoscopic apparatus 7 that is a modified example of the electronic endoscopic apparatus 6. In the shown example, the electronic endoscopic apparatus 7 includes a scope distal portion 210, an image processor portion 720, a scope cable portion 130, and a monitor 140. The image processor portion 720 includes a second multiplying/dividing circuit 121, an SSG 122, a data reception circuit 123, a synchronization frame memory 424, an image processing circuit 125, a display clock oscillator 701, a phase comparator 702, and a third multiplying/dividing circuit 703.

The display clock oscillator 701 is an oscillator such as a crystal oscillator, and generates a clock.
An imaging vertical synchronization signal transmitted from the scope distal portion 210 is input to the phase comparator 702. The phase comparator 702 compares a phase of the input imaging vertical synchronization signal with a phase of the clock output by the display clock oscillator 701. Then, the phase comparator 702 controls oscillation of the display clock oscillator 701 such that the phase of the clock output by the display clock oscillator 701 is identical to that of the imaging vertical synchronization signal. That is, the phase comparator 702 controls a frequency of the imaging clock output by the display clock oscillator 701.

The third multiplying/dividing circuit 703 multiples and/or divides the clock output by the display clock oscillator 701 by (natural number/natural number), generates a display clock having a frequency required to display an image, which is based on the image signal transmitted from the scope distal portion 310, on the monitor 140, and outputs the generated display clock to the SSG 122, the synchronization frame memory 424, and the image processing circuit 125. The other configurations of the electronic endoscopic apparatus 7 are similar to the configurations of the electronic endoscopic apparatus 6.

With this configuration, the clock oscillator 111 of the scope distal portion 210 outputs a master clock. Further, the first multiplying/dividing circuit 112 multiples and/or divides the master clock by (natural number/natural number), thereby generating an imaging clock and a transmission clock. Further, the transmission clock is transmitted to the second multiplying/dividing circuit 121 of the image processor portion 720. Also, the TG 213 generates the imaging vertical synchronization signal from the imaging clock. Further, the imaging vertical synchronization signal is transmitted to the synchronization frame memory 424 of the image processor portion 720. In addition, the imaging device 114 is operated by the imaging clock, captures a one-frame image at a timing based on the imaging vertical synchronization signal, and outputs image data.

On the other hand, the display clock oscillator 701 of the image processor portion 720 outputs a clock having a phase that is identical to that of the imaging vertical synchronization signal under control of the phase comparator 702. Further, the third multiplying/dividing circuit 703 multiples and/or divides the clock output by the display clock oscillator 701 by (natural number/natural number), thereby generating a display clock. Further, the SSG 122 generates a display vertical synchronization signal from the display clock generated by the third multiplying/dividing circuit 703. In addition, the image processing circuit 125 causes the monitor 140 to display the one-frame image at a timing based on the display vertical synchronization signal generated by the SSG 122.

Thereby, the clock output by the display clock oscillator 701 has the same phase as the imaging vertical synchronization signal. Further, the imaging vertical synchronization signal is generated based on the clock output by the display clock oscillator 701. Accordingly, the one-frame cycle for imaging can completely match that for displaying. Further, the imaging device 114 can capture the one-frame image at a timing based on the imaging vertical synchronization signal, and the image processing circuit 125 can cause the monitor 140 to display the one-frame image at a timing based on the display vertical synchronization signal. As such, by matching the cycle of the imaging vertical synchronization signal and the cycle of the display vertical synchronization signal, the electronic endoscopic apparatus 7 can secure the synchronization between the imaging and the displaying.

Accordingly, even when the processing of the imaging device 114 is performed at a high speed, the electronic endoscopic apparatus 7 can secure the synchronization between the cycle in which the scope distal portion 210 captures the image and the cycle in which the image processor portion 720 causes the monitor 140 to display the image. Thereby, the electronic endoscopic apparatus 7 can also suppress a phenomenon called "passing" or "frame dropping." Here, to simplify the description, description of timing control in which typical image processing such as correction, color conversion, filtering or the like is performed on the data (RAW) of the imaging device is omitted.

In this configuration, even when the imaging vertical synchronization signal and the display vertical synchronization signal are set to the same cycle, a structure in which the phase is maintained in a constant relation is not provided. Accordingly, a delay time from when the image is captured to when the image is displayed is altered depending on an application timing of power or an exchange timing of the scope. However, since the electronic endoscopic apparatus 7 continuously outputs operation of the display vertical synchronization signal, the synchronization between when the image is captured and when the image is displayed on the monitor 140 is not disturbed even when the scope distal portion 210 is exchanged during operation. Furthermore, in this configuration, since the scope distal portion 210 and the image processor portion 720 have the clock oscillator 111 and the display clock oscillator 701, respectively, even when the scope distal portion 210 and the image processor portion 720 are separated from each other, the image processor portion 720 can continue the corresponding operating, for instance, can continue to display the image on the monitor 140.

## Claims

1. An electronic endoscopic apparatus comprising:
an imaging device that is installed on a scope distal portion and captures an image based on an imaging clock;
an image processor portion that performs image processing on the image captured by the imaging device and displays the corresponding image on a monitor based on a display clock;
a scope cable portion that transmits data between the scope distal portion and the image processor portion;
a clock oscillator that generates a master clock; and
a multiplying/dividing circuit that multiplies and/or divides the master clock by (natural number/natural number) and generates a transmission clock whose frequency is lower than that of the imaging clock and is (natural number) times the frequency of a vertical synchronization signal.

2. The electronic endoscopic apparatus according to claim 1, further comprising a second multiplying/dividing circuit that multiplies and/or divides the transmission clock by (natural number/natural number) and generates a necessary frequency,
wherein the imaging clock or the display clock is generated from the master clock.

3. The electronic endoscopic apparatus according to claim 2, wherein the clock oscillator is installed on the scope distal portion,
the multiplying/dividing circuit is installed on the scope distal portion, and generates the imaging clock and the transmission clock, and
the second multiplying/dividing circuit is installed on the image processor portion, and generates the display clock.

4. The electronic endoscopic apparatus according to claim 3, wherein the multiplying/dividing circuit multiplies and/or divides the master clock by (natural number/natural number), and generates the imaging clock, and
the second multiplying/dividing circuit multiplies and/or divides the transmission clock by (natural number/natural number), and generates the display clock.

5. The electronic endoscopic apparatus according to claim 3, further comprising a display synchronization signal generating unit that generates a display vertical synchronization signal for displaying the image from the display clock,
wherein the scope cable portion transmits the transmission clock and the display vertical synchronization signal from the image processor portion to the scope distal portion.

6. The electronic endoscopic apparatus according to claim 5, further comprising an imaging synchronization signal generating unit that generates an imaging vertical synchronization signal for capturing the image from the imaging clock at a timing preceding a timing at which the display vertical synchronization signal is generated by at least a time that is the sum of a transmission delay time of the scope cable portion and a processing time at the image processor portion.

7. The electronic endoscopic apparatus according to claim 3, further comprising an imaging synchronization signal generating unit that generates an imaging vertical synchronization signal for capturing the image from the imaging clock,
wherein the scope cable portion transmits the transmission clock and the imaging vertical synchronization signal from the scope distal portion to the image processor portion.

8. The electronic endoscopic apparatus according to claim 7, further comprising a display synchronization signal generating unit that generates a display vertical synchronization signal for displaying the image from the display clock at a timing delaying from a timing at which the imaging vertical synchronization signal is generated by at least a time that is the sum of a transmission delay time of the scope cable portion and a processing time at the image processor portion.

9. The electronic endoscopic apparatus according to claim 2, wherein the clock oscillator is installed on the image processor portion,
the multiplying/dividing circuit is installed on the image processor portion, and generates the display clock and the transmission clock, and
the second multiplying/dividing circuit is installed on the scope distal portion, and generates the imaging clock.

10. The electronic endoscopic apparatus according to claim 9, wherein the multiplying/dividing circuit multiplies and/or divides the master clock by (natural number/natural number), and generates the display clock, and
the second multiplying/dividing circuit multiplies and/or divides the transmission clock by (natural number/natural number), and generates the imaging clock.

11. The electronic endoscopic apparatus according to claim 9, further comprising a display synchronization signal generating unit that generates a display vertical synchronization signal for displaying the image from the display clock,
wherein the scope cable portion transmits the transmission clock and the display vertical synchronization signal from the image processor portion to the scope distal portion.

12. The electronic endoscopic apparatus according to claim 11, further comprising an imaging synchronization signal generating unit that generates an imaging vertical synchronization signal for capturing the image from the imaging clock at a timing preceding a timing at which the display vertical synchronization signal is generated by at least a time that is the sum of a transmission delay time of the scope cable portion and a processing time at the image processor portion.

13. The electronic endoscopic apparatus according to claim 9, further comprising an imaging synchronization signal generating unit that generates an imaging vertical synchronization signal for capturing the image from the imaging clock,
wherein the scope cable portion transmits the transmission clock and the imaging vertical synchronization signal from the scope distal portion to the image processor portion.

14. The electronic endoscopic apparatus according to claim 13, further comprising a display synchronization signal generating unit that generates a display vertical synchronization signal for displaying the image from the display clock at a timing delaying from a timing at which the imaging vertical synchronization signal is generated by at least a time that is the sum of a transmission delay time of the scope cable portion and a processing time at the image processor portion.

15. The electronic endoscopic apparatus according to claim 7 or 13, wherein the imaging vertical synchronization signal and the display vertical synchronization signal are generated so as to have the same frequency.
